(19) Europäisches Patentamt
European Patent Office
Office européen des brevets

(11) Veröffentlichungsnummer: **0 035 084**
**B1**

(12) # EUROPÄISCHE PATENTSCHRIFT

(45) Veröffentlichungstag der Patentschrift: **31.08.83**

(51) Int. Cl.³: **C 07 C 127/22**, A 01 N 47/34

(21) Anmeldenummer: **80810082.0**

(22) Anmeldetag: **05.03.80**

(54) **Substituierte N-Fluorphenyl-N'-halogenbenzoylharnstoffe, Verfahren zu ihrer Herstellung, diese Verbindungen enthaltende Mittel und ihre Verwendung zur Bekämpfung von Schädlingen.**

(43) Veröffentlichungstag der Anmeldung: **09.09.81 Patentblatt 81/36**

(45) Bekanntmachung des Hinweises auf die Patenterteilung: **31.08.83 Patentblatt 83/35**

(84) Benannte Vertragsstaaten: **AT BE FR GB IT NL**

(56) Entgegenhaltungen: **GB-A-1 221 116**

(73) Patentinhaber: **CIBA-GEIGY AG, Patentabteilung Postfach, CH-4002 Basel (CH)**

(72) Erfinder: **Ehrenfreund, Josef, Dr., Langenhagweg 11, CH-4123 Allschwil (CH)**

EP 0 035 084 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Substituierte N-Fluorphenyl-N'-halogenbenzoylharnstoffe, Verfahren zu ihrer Herstellung, diese Verbindungen enthaltende Mittel und ihre Verwendung zur Bekämpfung von Schädlingen.

Die vorliegende Erfindung betrifft neue N-Tetrafluorphenyl-N'-halogenbenzoylharnstoffe, Verfahren zu ihrer Herstellung und ihre Verwendung in der Schädlingsbekämpfung.

Die erfindungsgemässen substituierten N-Tetrafluorphenyl-N'-halogenbenzoylharnstoffe haben die Formel I

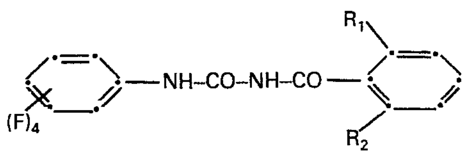

(I) ,

worin $R_1$ Fluor oder Chlor und $R_2$ Wasserstoff, Fluor oder Chlor bedeuten.

Wegen ihrer Wirkung als Schädlingsbekämpfungsmittel bevorzugt sind erfindungsgemässe Verbindungen der Formel Ia.

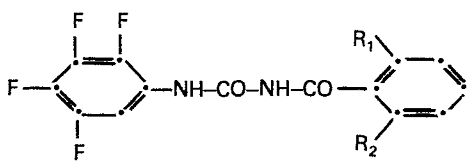

(Ia),

worin $R_1$ und $R_2$ die oben angegebene Bedeutung besitzen.

Aufgrund ihrer guten insektiziden Wirksamkeit ist die erfindungsgemässe Verbindung der Formel

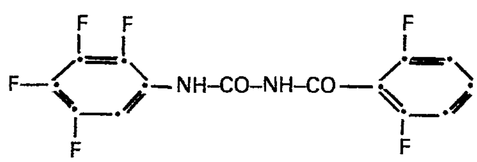

besonders hervorzuheben.

Die Verbindungen der Formel I können nach an sich bekannten Verfahren hergestellt werden (vgl. u.a. die deutschen Offenlegungsschriften Nr. 2 123 236, 2 601 780).

So kann man z.B. eine Verbindung der Formel I erhalten durch Umsetzung

a) einer Verbindung der Formel II

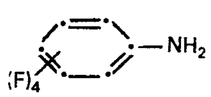

(II)

mit einer Verbindung der Formel III

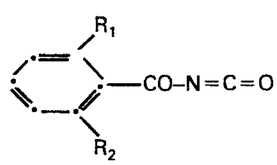

(III)

oder

b) einer Verbindung der Formel IV

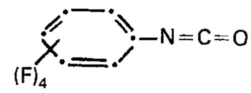

(IV)

gegebenenfalls in Gegenwart einer basischen Substanz mit einer Verbindung der Formel V

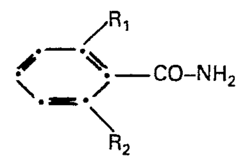

(V)

In den obigen Formeln II, III, IV und V haben die Reste $R_1$ und $R_2$ die unter Formel I und Ia vorstehend angegebenen Bedeutungen.

Die erwähnten Verfahren a) und b) können vorzugsweise unter normalem Druck und in Gegenwart eines organischen Lösungs- oder Verdünnungsmittels durchgeführt werden. Als Lösungs- oder Verdünnungsmittel eignen sich z.B. Äther und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; Dimethylsulfoxid sowie Ketone, z.B. Aceton, Methyläthylketon, Methylisopropylketon und Methylisobutylketon. Verfahren a) wird im allgemeinen bei einer Temperatur von −10 bis 100 °C, vorzugsweise zwischen 15 und 25 °C, gegebenenfalls in Gegenwart einer organischen Base, z.B. Triäthylamin, durchgeführt. Die Durchführung von Verfahren b) erfolgt bei einer Temperatur von 0 bis 150 °C, vorzugsweise beim Siedepunkt des verwendeten Lösungsmittels, und gegebenenfalls in Gegenwart einer organischen Base, wie Pyridin, und/oder unter Zusatz eines Alkali- oder Erdalkalimetalls, vorzugsweise Natrium.

Die Ausgangsstoffe der Formeln II, III, IV und V sind bekannt oder können analog bekannten Verfahren hergestellt werden. Beispielsweise sind die Tetrafluorisocyanate der Formel IV durch Phosgenisierung der entsprechenden Tetrafluoraniline der Formel II nach allgemein üblichen Verfahren erhältlich.

Es ist bereits bekannt, dass bestimmte N-Phenyl-N'-benzoylharnstoffe insektizide Eigenschaften besitzen (vgl. deutsche Offenlegungsschriften 2 123 236, 2 504 982, 2 531 279, 2 537 413, 2 601 780 und 2 726 684 sowie die belgischen Patentschriften 832 304, 843 906 und 844 066).

Überraschenderweise wurde nun gefunden, dass die erfindungsgemässen N-Tetrafluorphenyl-N' -halogenbenzoylharnstoffe der Formel I bei guter Pflanzenverträglichkeit und geringer Warmblütertoxizität ausgezeichnete Wirksamkeit als Schädlingsbekämpfungsmittel aufweisen.

Sie eignen sich vor allem zur Bekämpfung von Pflanzen und Tiere befallenden Schädlingen.

Insbesondere eignen sich die Verbindungen der Formel I zur Bekämpfung von Insekten der Ordnungen: Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera.

Wegen ihrer sehr günstigen Wirkung als ovizide und ovolarvizide Wirkstoffe eignen sich die Verbindungen der Formel I auch zur Bekämpfung von pflanzenschädigenden Frassinsekten in Zier- und Nutzpflanzungen, insbesondere in Baumwollkulturen (z.B. gegen Spodoptera littoralis und Heliothis virescens) sowie in Gemüsekulturen (z.B. gegen Leptinotarsa decemlineata). Die Verbindungen der Formel I zeichnen sich durch eine ausgeprägte Wirkung gegen larvale Insektenstadien und Insekteneier, insbesondere gegen larvale Stadien und Eier fressender Schadinsekten, aus. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder eine reduzierte Schlupfrate festzustellen.

Die Verbindungen der Formel I eignen sich weiterhin zur Bekämpfung von Ectoparasiten an Haus- und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden wesentlich verbreitern und an gegebene Umstände anpassen.

Als Zusätze kommen z.B. folgende Wirkstoffe in Betracht: organische Phosphorverbindungen, Nitrophenole und Derivaten, Formamidine, Harnstoffe, Carbamate und chlorierte Kohlenwasserstoffe.

Mit besonderem Vorteil kann man die Verbindungen der Formel I auch mit Substanzen kombinieren, welche einen pestizid verstärkenden Effekt ausüben. Beispiele solcher Verbindungen sind u.a.: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I können für sich allein oder zusammen mit geeigneten Trägern und/oder Zuschlagstoffen eingesetzt werden. Geeignete Träger und Zuschlagstoffe können fest oder flüssig sein und entsprechen den in der Formulierungstechnik üblichen Stoffen, wie z.B. natürlichen oder regenerierten Stoffen, Lösungs-, Dispergier-, Netz-, Haft-, Verdickungs-, Binde- und/oder Düngemitteln. Zur Applikation können die Verbindungen der Formel I zu Stäubemitteln, Emulsionskonzentraten, Granulaten, Dispersionen, Sprays, zu Lösungen oder Aufschlämmungen in üblicher Formulierung, die in der Applikationstechnik zum Allgemeinwissen gehören, verarbeitet werden. Ferner sind «cattle dips», d.h. Viehbäder, und «spray races», d.h. Sprühgänge, in denen wässrige Zubereitungen verwendet werden, zu erwähnen. Diese Zubereitungsformen sind insbesondere zur Bekämpfung tierparasitärer Schädlinge geeignet.

Die Herstellung erfindungsgemässer Mittel erfolgt in an sich bekannter Weise durch inniges Vermischen und/oder Vermahlen von Wirkstoffen der Formel I, mit geeigneten Trägerstoffen, gegebenenfalls unter Zusatz von gegenüber den Wirkstoffen inerten Dispergier- und Lösungsmitteln. Die Wirkstoffe können in den folgenden Aufarbeitungsformen vorliegen und angewendet werden:

Feste Aufarbeitungsformen:
Stäubemittel, Streumittel, Granulate (Umhüllungsgranulate, Imprägnierungsgranulate und Homogengranulate);
Flüssige Aufarbeitungsformen:
a) in Wasser dispergierbare Wirkstoffkonzentrate:
Spritzpulver (wettable powders), Pasten, Emulsionen;
b) Lösungen.

Der Gehalt an Wirkstoff in den oben beschriebenen Mitteln liegt zwischen 0,1 bis 95%.

Die Wirkstoffe der Formel I können beispielsweise wie folgt formuliert werden:

Stäubemittel:

Zur Herstellung eines a) 5%igen und b) 2%igen Stäubemittels werden die folgenden Stoffe verwendet:

a) 5 Teile Wirkstoff
95 Teile Talkum;
b) 2 Teile Wirkstoff
1 Teil hochdisperse Kieselsäure
97 Teile Talkum.

Der Wirkstoff wird mit den Trägerstoffen vermischt und vermahlen.

Granulate:

Zur Herstellung eines 5%igen Granulates werden die folgenden Stoffe verwendet:

5 Teile Wirkstoff
0,25 Teile epoxidiertes Pflanzenöl
0,25 Teile Cetylpolyglykoläther
3,50 Teile Polyäthylenglykol
91 Teile Kaolin (Korngrösse 0,3–0,8 mm).

Die Aktivsubstanz wird mit Epichlorhydrin vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

Spritzpulver:

Zur Herstellung eines a) 40%igen, b) und c) 25%igen, d) 10%igen Spritzpulvers werden folgende Bestandteile verwendet:

a) 40 Teile Wirkstoff
5 Teile Ligninsulfonsäure-Natriumsalz
1 Teil Dibutylnaphthalinsulfonsäure-Natriumsalz
54 Teile Kieselsäure;

b) 25 Teile Wirkstoff
4,5 Teile Calcium-Ligninsulfonat
1,9 Teile Champagne-Kreide Hydroxyäthylcellulose Gemisch (1:1)
1,5 Teile Natrium-dibutyl-naphthalinsulfonat
19,5 Teile Kieselsäure
19,5 Teile Champagne-Kreide
28,1 Teile Kaolin;

c) 25 Teile Wirkstoff
2,5 Teile Isooctylphenoxy-polyäthylen-äthanol
1,7 Teile Champagne-Kreide Hydroxyäthylcellulose-Gemisch (1:1)
8,3 Teile Natriumaluminiumsilikat
16,5 Teile Kieselgur
46 Teile Kaolin;

d) 10 Teile Wirkstoff
3 Teile Gemisch der Natriumsalze von gesättigten Fettalkoholsulfaten
5 Teile Naphthalinsulfonsäure Formaldehyd-Kondensat
82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate:

Zur Herstellung eines a) 10%igen, b) 25%igen und c) 50%igen emulgierbaren Konzentrates werden folgende Stoffe verwendet:

a) 10 Teile Wirkstoff
3,4 Teile epoxidiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol;

b) 25 Teile Wirkstoff
2,5 Teile epoxydiertes Pflanzenöl
10 Teile eines Alkylarylsulfonat Fettalkoholpolyglykoläther-Gemisches
5 Teile Dimethylformamid
57,5 Teile Xylol;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentraten können durch Verdünnen mit Wasser Emulsionen jeder gewünschten Konzentration hergestellt werden.

Sprühmittel:

Zur Herstellung eines a) 5%igen und b) 95%igen Sprühmittels werden die folgenden Bestandteile verwendet:

a) 5 Teile Wirkstoff
1 Teil epoxydiertes Pflanzenöl
94 Teile Benzin (Siedegrenzen 160–190 °C);

b) 95 Teile Wirkstoff
5 Teile epoxydiertes Pflanzenöl

Beispiel 1

Zu einer Lösung von 4,95 g 2,3,4,5-Tetrafluoranilin in 20 ml absolutem Äther werden bei Raumtemperatur und unter Ausschluss von Feuchtigkeit 5,8 g 2,6-Difluorbenzoylisocyanat zugesetzt. Der nach kurzer Zeit ausfallende Niederschlag wird absaugt; durch Umkristallisation aus Toluol erhält man N-2,3,4,5-Tetrafluorphenyl-N'-2,6-difluorbenzoylharnstoff vom Schmelzpunkt 198–202 °C.

Beispiel 2

Es werden 4,95 g 2,3,4,6-Tetrafluoranilin in 20 ml absolutem Äther gelöst und die Lösung bei Raumtemperatur und unter Ausschluss von Feuchtigkeit mit 5,8 g 2,6-Difluorbenzoylisocyanat versetzt. Der ausgefallene Niederschlag wird abgesauft und aus Toluol umkristallisiert. Man erhält N-2,3,4,6-Tetrafluorphenyl-N'-2,6-difluorbenzoylharnstoff vom Schmelzpunkt 178–182 °C.

Beispiel 3

Analog den vorstehend beschriebenen Arbeitsweisen werden auch die folgenden Verbindungen der Formel I hergestellt:

| $(F)_4$ Stellung der F-Substituenten | $R_1$ | $R_2$ | Schmelzpunkt [°C] |
|---|---|---|---|
| 2.3.4.5 | Cl | Cl | 215–217 |
| 2.3.4.5 | Cl | H | 199–206 |
| 2.3.4.5 | F | H | 179.5–181 |
| 2.3.4.5 | F | Cl | 213–215 |
| 2.3.4.6 | Cl | Cl | 179–182 |
| 2.3.4.6 | Cl | H | 173–176 |
| 2.3.4.6 | F | H | 152–155 |
| 2.3.4.6 | F | Cl | 179–181 |
| 2.3.5.6 | F | F | 199–200 |
| 2.3.5.6 | Cl | Cl | 182–185 |
| 2.3.5.6 | Cl | H | 174–177 |
| 2.3.5.6 | F | H | 138–145 |
| 2.3.5.6 | F | Cl | 190–194 |

Beispiel 4

Wirkung gegen Musca domestica:

Je 50 frisch zubereitetes CSMA-Nährsubstrat für Maden wurde in Becher eingewogen. Von einer 1 Gew.-%igen acetonischen Lösung des betreffenden Wirkstoffes wurde eine bestimmte Menge auf das in den Bechern befindliche Nährsubstrat pipettiert. Nach dem Durchmischen des Substrates lässt man das Aceton mindestens 20 Stunden lang verdampfen.

Dann wurden pro Wirkstoff und Konzentration je 25 eintägige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmte Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Verbindungen gemäss den Beispielen 1–3 zeigten gute Wirkung im obigen Test.

Beispiel 5
Wirkung gegen Aëdes aegypti:

Auf die Oberfläche von 150 ml Wasser, das sich in einem Behälter befindet, wurde so viel einer 0,1%igen acetonischen Lösung des Wirkstoffes pipettiert, dass Konzentrationen von je 10,5 und 1 ppm erhalten wurden. Nach Verdunsten des Acetons wurde der Behälter mit 30–40 3-tägigen Aëdes-Larven beschickt. Nach 1, 2 und 5 Tagen wurde die Mortalität geprüft.

Verbindungen gemäss den Beispielen 1 bis 3 zeigten in diesem Test gute Wirkung gegen Aëdes aegypti.

Beispiel 6
Ovizide Wirkung auf Heliothis virescens und Spodoptera littoralis:

Entsprechende Mengenanteile einer benetzbaren pulverförmigen Formulierung, enthaltend 25 Gew.-% des zu prüfenden Wirkstoffes, wurden mit jeweils soviel Wasser verdünnt, dass sich wässrige Emulsionen von ansteigender Wirkstoffkonzentration ergaben.

In diese wirkstoffhaltigen Emulsionen wurden eintägige, auf Fliesspapier abgelegte Eigelege von Heliothis bzw. Spodoptera während drei Minuten eingetaucht und dann auf Rundfiltern abgenutscht. Die so behandelten Gelege wurden in Petrischalen ausgelegt und in der Dunkelheit aufbewahrt. Nach 6 bis 8 Tagen wurde die Schlupfrate im Vergleich zu unbehandelten Kontrollen festgestellt. Zur Auswertung wurde die zur 100%-igen Abtötung der Eier erforderlich minimale Wirkstoffkonzentration bestimmt.

Die Verbindungen gemäss den Beispielen 1 bis 3 zeigten in diesem Test gute ovizide Wirkung gegen die geprüften Schädlinge.

Beispiel 7
Wirkung gegen Lucilia sericata:

Zu 9 ml eines Zuchtmediums wurde bei 50 °C 1 ml einer 0,5% Aktivsubstanz enthaltenden wässrigen Zubereitung gegeben. Nun wurden ca. 30 frisch geschlüpfte Lucilia sericata-Larven zum Zuchtmedium gegeben und nach 48 und 96 Stunden die insektizide Wirkung durch Ermittlung der Abtötungsrate festgestellt.

Verbindungen gemäss den Beispielen 1 bis 3 zeigten in diesem Test gute Wirkung gegen Lucilia sericata.

Beispiel 8
Wirkung auf Spodoptera littoralis (Adulte):

Gefässe mit einem Volumen von 20 Litern enthaltend jeweils drei ca. 30 cm hohe Baumwoll-Pflanzen wurden mit einer Lösung des zu prüfenden Wirkstoffes besprüht, bis die Lösung von den Pflanzen abtropfte. Die so behandelten Pflanzen wurden 12 Tage bei 30–35 °C und 60–70% relativer Luftfeuchtigkeit gehalten. Nach Ablauf dieses Zeitraumes wurden die Gefässe mit je 10 zwei- bis dreitägigen adulten Weibchen von Spodoptera belegt und für zwei Tage bei 28 °C und 60% relativer Luftfeuchtigkeit gehalten.

Danach erfolgte Auswertung hinsichtlich der Mortalität der adulten Falter, der Anzahl der abgelegten Eier sowie des Frasses geschlüpfter Larven im Vergleich zu unbehandelten Kontrollen.

Verbindungen gemäss den Beispielen 1 bis 3 zeigten gute Wirkung in diesem Test.

Beispiel 9
Wirkung gegen Spodoptera littoralis und Heliothis virescens (Larven; Frass- und Kontaktwirkung):

Eingetopfte ca. 30 cm hohe Baumwoll- bzw. Soja-Pflanzen wurden mit einer verdünnten, wässrigen Emulsions-Zubereitung des zu prüfenden Wirkstoffes bis zum Abtropfen besprüht. Nach Antrocknen des Spritzbelages wurden die Baumwoll-Pflanzen mit je 5 Larven im dritten Larvalstadium von Spodoptera und die Soja-Pflanzen mit je 10 Larven im dritten Larvalstadium von Heliothis besetzt. Die Ansätze wurden 5 Tage bei künstlichem Licht, einer Temperatur von etwa 26 °C und 50–60% relativer Luftfeuchtigkeit gehalten.

Die Auswertung erfolgte hinsichtlich prozentualer Mortalität, Frasshemmung, Deformationen und Entwicklungshemmungen im Vergleich zu unbehandelten Kontrollen.

Die Verbindungen gemäss den Beispielen 1 bis 3 zeigten in diesem Test gute Wirkung.

Beispiel 10
Wirkung auf Spodoptera littoralis und Heliothis virescens (Larven und Eier):

Es wurden drei in Töpfen gezogene Baumwollpflanzen von ca. 15–20 cm Höhe mit einer sprühfähigen flüssigen Zubereitung des zu prüfenden Wirkstoffes behandelt. Nach Antrocknen des Sprühbelages wurden die eingetopften Pflanzen in ein Blechgefäss von etwa 20 Litern Inhalt gestellt, das mit einer Glasplatte abgedeckt wurde. Die Feuchtigkeit im Inneren des abgedeckten Gefässes wurde so reguliert, dass sich kein Kondenswasser bildete. Direktes, auf die Pflanzen fallendes Licht wurde vermieden. Dann wurden die drei Pflanzen infestiert, und zwar insgesamt mit:

a) 50 Larven von Spodoptera littoralis oder Heliothis virescens des ersten larvalen Stadiums;
b) 20 Larven von Spodoptera litteralis oder Heliothis virescens des dritten larvalen Stadiums;
c) zwei Eispiegeln von Spodoptera littoralis oder Heliothis virescens. Dazu wurden je 2 Blätter einer Pflanze in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis wurden zu den eingeschlossenen Blättern gegeben.

Nach 4 bis 5 Tagen erfolgte die Auswertung gegenüber unbehandelten Kontrollen unter Berücksichtigung folgender Kriterien:

a) Anzahl der noch lebenden Larven,
b) Larvale Entwicklungs- und Häutungshemmung,
c) Frasschaden (Schabfrass und Lochfrass),
d) Schlupfrate (Anzahl der aus den Eiern geschlüpften Larven).

Die Verbindungen gemäss den Beispielen 1 bis 3 zeigten gute Gesamt-Wirksamkeit in obigem Test.

Beispiel 11
Chemosterilisierende Wirkung auf Anthonomus grandis.

Adulte Anthonomus grandis, die nach dem Schlupf nicht älter als 24 Stunden waren, wurden in Gruppen zu jeweils 25 Käfern in Käfige mit Gitterwänden überführt. Die mit den Käfern besetzten Käfige wurden sodann während 5 bis 10 Sekunden in eine acetonische Lösung, enthaltend 1,0 Gew.-% des zu prüfenden Wirkstoffes, eingetaucht.

Nachdem die Käfer wieder trocken waren, wurden sie zur Kopulation und Eiablage in abgedeckte und Futter enthaltende Schalen eingesetzt. Abgelegte Eier wurden zwei- bis dreimal wöchentlich mit fliessendem Wasser ausgeschwemmt, gezählt, durch zwei- bis dreistündiges Einlegen in ein wässriges Desinfektionsmittel (wie z.B. «Actamer B 100») desinfiziert und dann in Schalen, die eine geeignete Larvaldiät enthielten, deponiert. Nach 7 Tagen wurde untersucht, ob sich aus den deponierten Eiern Larven entwickelt hatten.

Zur Ermittlung der Dauer des Chemosterilans-Effektes der zu prüfenden Wirkstoffe wurde die Eiablage der Käfer während eines Zeitraumes von etwa vier Wochen überprüft. Die Bonitierung erfolgte anhand der Verminderung der Anzahl abgelegter Eier und geschlüpfter Larven im Vergleich zu unbehandelten Kontrollen.

Verbindungen gemäss den Beispielen 1 bis 3 zeigten gute Wirkung in obigem Test.

**Patentansprüche**

1. Verbindung der Formel I

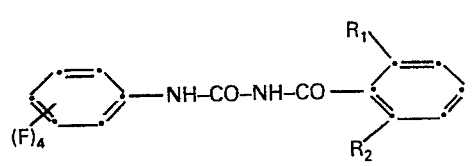

(I) ,

worin $R_1$ Fluor oder Chlor und $R_2$ Wasserstoff, Fluor oder Chlor bedeuten.

2. Verbindung gemäss Anspruch 1 der Formel Ia

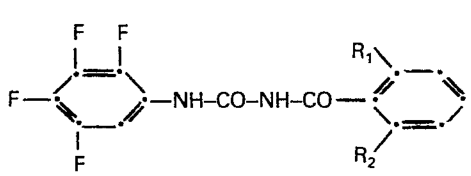

(Ia),

worin $R_1$ und $R_2$ die unter Anspruch 1 angegebenen Bedeutungen haben.

3. Verbindung gemäss Anspruch 2 der Formel

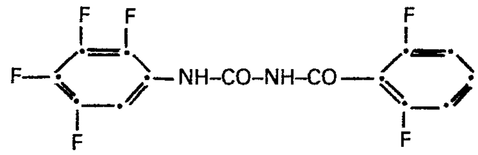

4. Verbindung gemäss Anspruch 2 der Formel

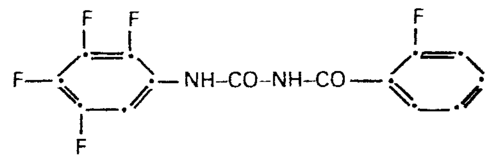

5. Verbindung gemäss Anspruch 2 der Formel

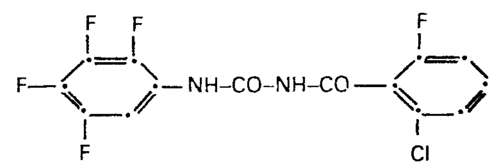

6. Verbindung gemäss Anspruch 1 der Formel

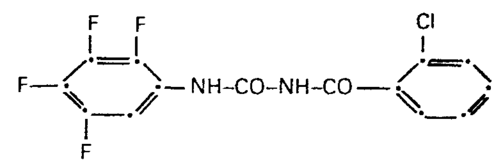

7. Verfahren zur Herstellung einer Verbindung gemäss den Ansprüchen 1 bis 6, dadurch gekennzeichnet, dass man

a) eine Verbindung der Formel II

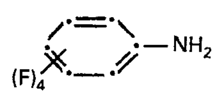

(II)

mit einer Verbindung der Formel III

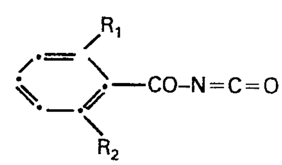

(III)

oder

b) eine Verbindung der Formel IV

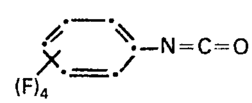

(IV)

mit einer Verbindung der Formel V

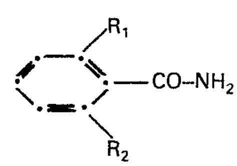

(V)

umsetzt, wobei in den Formeln III und V die Reste $R_1$ und $R_2$ die unter Anspruch 1 und 2 angegebenen Bedeutungen haben.

8. Schädlingsbekämpfungsmittel, welches als aktive Komponente eine Verbindung gemäss den Ansprüchen 1 bis 6 zusammen mit geeigneten Träger- und/oder anderen Zuschlagstoffen enthält.

9. Verwendung einer Verbindung gemäss den Ansprüchen 1 bis 6 zur Bekämpfung von Schädlingen, vorzugsweise von Insekten.

10. Verwendung nach Anspruch 9 einer Verbindung gemäss den Ansprüchen 1 bis 6 als Ovizid, Ovolarvizid oder als Chemosterilans.

**Revendications**

1. Composé de formule I

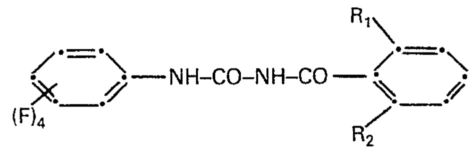

(I),

dans laquelle $R_1$ représente le fluor ou le chlore et $R_2$ l'hydrogène, le fluor ou le chlore.

2. Composé selon la revendication 1, répondant à la formule Ia

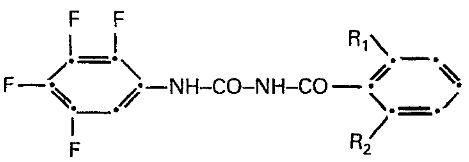

(Ia),

dans laquelle $R_1$ et $R_2$ ont les significations indiquées dans la revendication 1.

3. Composé selon la revendication 2, répondant à la formule

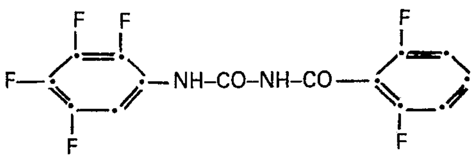

4. Composé selon la revendication 2, répondant à la formule

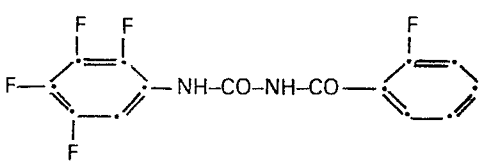

5. Composé selon la revendication 2, répondant à la formule

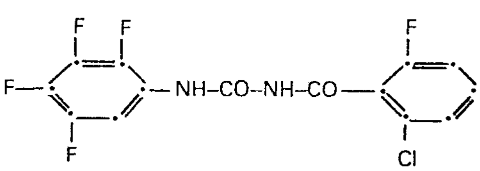

6. Composé selon la revendication 1, répondant à la formule

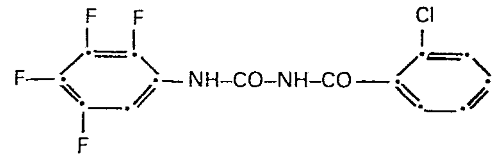

7. Procédé de préparation d'un composé selon les revendications 1 à 6, caractérisé en ce que:

a) on fait réagir un composé de formule II

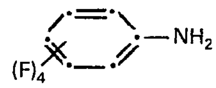

(II)

avec un composé de formule III

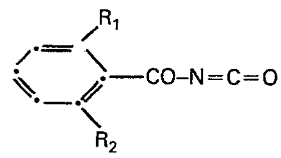

(III)

ou bien

b) on fait réagir un composé de formule IV

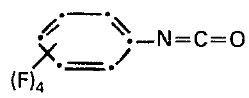

(IV)

avec un composé de formule V

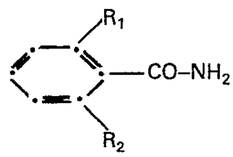

(V)

les symboles $R_1$ et $R_2$ ayant dans les formules III et V les significations indiquées dans les revendications 1 et 2.

8. Produit pesticide contenant en tant que composant actif un composé selon les revendications 1 à 6 avec des véhicules et/ou d'autres additifs appropriés.

9. Utilisation d'un composé selon les revendications 1 à 6, dans la lutte contre les parasites, de préférence les insectes.

10. Utilisation selon la revendication 9 d'un composé selon les revendications 1 à 6 en tant qu'ovicide, ovolarvicide ou chimiostérilisant.

**Claims**

1. A compound of the formula

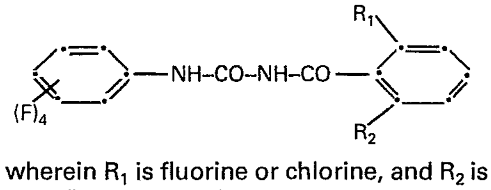

(I),

wherein $R_1$ is fluorine or chlorine, and $R_2$ is hydrogen, fluorine or chlorine.

2. A compound according to Claim 1 of the formula Ia

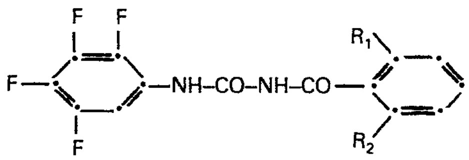

(Ia),

wherein $R_1$ and $R_2$ have the meanings given under Claim 1.

3. A compound according to Claim 2 of the formula

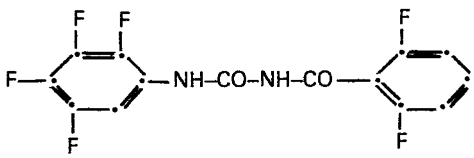

4. A compound according to Claim 2 of the formula

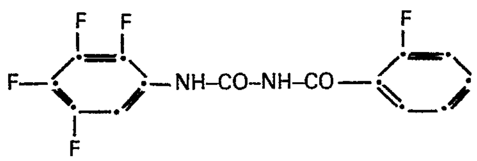

5. A compound according to Claim 2 of the formula

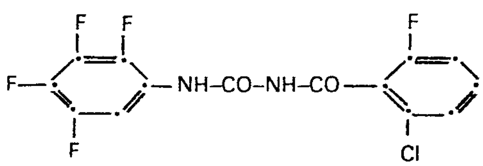

6. A compound according to Claim 1 of the formula

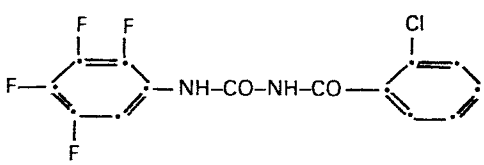

7. A process for producing a compound according to Claims 1 to 6 inclusive, which process coprises reacting

a) a compound of the formula II

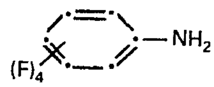

(II)

with a compound of the formula III

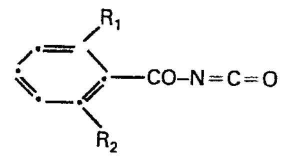

(III)

or

b) a compound of the formula IV

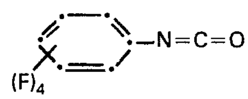

(IV)

with a compound of the formula V

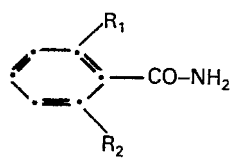

(V)

wherein in the formulae III and V, the radicals $R_1$ and $R_2$ have the meanings given under Claims 1 and 2.

8. A pesticidal composition which contains as active ingredient a compound according to Claims 1 to 6 inclusive, together with suitable carriers and/or other additives.

9. Use of a compound according to Claims 1 to 6 for combating pests, particularly insects.

10. Use according to Calim 9 of a compound according to Claims 1 to 6 as an ovicide or ovolarvicide, or as a chemosterilant.